Europäisches Patentamt

European Patent Office

Office européen des brevets

(19)

(11) Veröffentlichungsnummer: **0 011 143**
**B1**

(12) ## EUROPÄISCHE PATENTSCHRIFT

(45) Veröffentlichungstag der Patentschrift:
14.03.84

(51) Int. Cl.³: **A 61 F 5/47**

(21) Anmeldenummer: **79103977.9**

(22) Anmeldetag: **15.10.79**

(54) Vorrichtung zur intrauterinen Empfängnisverhütung.

(30) Priorität: **16.10.78 IT 8494278**

(43) Veröffentlichungstag der Anmeldung:
**28.05.80 Patentblatt 80/11**

(45) Bekanntmachung des Hinweises auf die Patenterteilung:
**14.03.84 Patentblatt 84/11**

(84) Benannte Vertragsstaaten:
**AT BE DE FR GB LU NL**

(56) Entgegenhaltungen:
**DE - C - 549 994**
**US - A - 3 291 125**
**US - A - 3 364 927**
**US - A - 3 438 369**
**US - A - 3 467 088**
**US - A - 3 507 274**
**US - A - 3 678 927**
**US - A - 3 811 435**
**US - A - 3 937 217**
**US - A - 4 117 838**

(73) Patentinhaber: **I.R.MED. S.r.l.(Istituto Ricerche Mediche), 6, Via del Casalino, CAP 37127 Verona (IT)**

(72) Erfinder: **Danieletto, Giuseppina, 24, Via Isonzo, CAP 37126 Verona (IT)**

(74) Vertreter: **Westphal, Klaus, Dipl.-Ing. et al, Patentanwälte Dipl.-Ing. Klaus Westphal Dr.rer.nat. Bernd Mussgnug Dr.rer.nat. Otto Buchner Flossmannstrasse 30a, D-8000 München 60 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Vorrichtung zur intrauterinen Empfängnisverhütung

Die Erfindung betrifft eine Vorrichtung zur intrauterinen Empfängnisverhütung, mit einem aus einem elastischen Kunststoff bestehenden, Y-förmigen Gerät, das einen eine Mittelachse besitzenden Halter und zwei von diesem ausgehende, bogenförmig aufgespreizte Zweige aufweist, die je eine Kupferdrahtwicklung tragen und in einem verdickten Kopf enden, und mit einem Auswerfer, der ein Rohr und einen im Rohr teleskopartig verschiebbaren, an einem Ende einen Handgriff tragenden Kolben aufweist, wobei das Gerät wenigstens teilweise in das Rohr einführbar ist, das Rohr schwergängig auf dem Kolben sitzt und auf seiner Außenseite einen Anschlagflansch trägt und das Rohr elastisch verformbar ist.

Eine Vorrichtung dieser Art ist aus der US-A-3 678 927 bekannt. Üblicherweise werden auf den Zweigen des Geräts Kupferdrahtwicklungen angebracht, die eine empfängnisverhütende Wirkung in den oberen Winkeln der Uterushöhlung nahe den Mündungen der Falloppioschen Kanäle ausüben. Bei dieser bekannten Vorrichtung besteht die Gefahr, daß beim Einführen und Benutzen des Geräts Schädigungen des Uterus, wie Perforationen, Metrorrhagien, Spotting, durch Reibung verursachte Reizungen und Entzündungen und dadurch bedingte Leiden, hervorgerufen werden. Bei einer Ausführungsform ist das bekannte Gerät zwar im Bereich der Halterung der beiden Zweige in Richtung seiner Mittelachse elastisch verformbar. Dadurch ergibt sich aber auch eine relativ große seitliche Elastizität der beiden Zweige, so daß das in den Uterushohlraum eingebrachte Gerät in seiner Lage nicht ausreichend gesichert ist und leicht infolge von Uteruskontraktionen unbeabsichtigt aus dem Uterus ausgestoßen werden kann.

Durch die Erfindung soll eine Vorrichtung zur intrauterinen Empfängnisverhütung geschaffen werden, die völlig sicher arbeitet, d. h. die weder beim Einbringen noch bei Benutzung des Geräts Anlaß zu Schädigungen des Uterus gibt und bei der das in die Uterushöhlung eingebrachte Gerät nicht unbeabsichtigt aus dem Uterus ausgestoßen werden kann.

Diese Aufgabe wird bei einem Gerät der eingangs genannten Art erfindungsgemäß dadurch gelöst, daß der Halter in Richtung seiner Mittelachse elastisch verformbar ist, indem er in sich mindestens zwei bezüglich der Mittelachse elastisch ausbiegbare Arme aufweist, die zusammen eine Raute bilden, deren eine Diagonale mit der Mittelachse zusammenfällt, daß jeder Zweig mindestens eine die Aufspreizstellung stabilisierende Verstärkungsrippe trägt und daß die beiden Köpfe formschlüssig ineinanderfügbare Teile aufweisen.

Die Ausbildung eines Abschnittes des Halters in Form einer geschlossenen Raute, die von ausbiegbaren Armen gebildet wird, gewährleistet ein sicheres Nachgeben des Geräts in Axialrichtung beim Auftreffen auf Uteruswandungen, beeinträchtigt jedoch andererseits nicht die Stabilität der am Halter sitzenden aufgespreizten Zweige des Geräts, die im übrigen von einer Bewegung der in den Halter eingearbeiteten Raute vollständig unabhängig sind. Die an den Zweigen vorgesehenen Rippen stabilisieren überdies deren Aufspreizstellung derart, daß einem unbeabsichtigten Ausstoßen des Geräts bei Uteruskontraktionen entgegengewirkt wird. Durch die Stabilisierung der Aufspreizstellung der Zweige mittels Verstärkungsrippen wird zudem verhindert, daß bei Druckausübung auf die freien Enden der Zweige ohne weiteres eine Vergrößerung ihrer Krümmungsradien eintritt und dadurch ein Herausgleiten des Geräts aus dem Uterus erleichtert wird. Durch die formschlüssige Halterung der beiden Köpfe wird beim Einführen des Geräts eine Verschiebung derselben und somit die Gefahr der Verletzung der Uteruswände sicher vermieden. Gleichzeitig wird durch die Schwergängigkeit zwischen Rohr und Kolben gewährleistet, daß der Kolben nicht unbeabsichtigt das Gerät aus dem Rohr ausstößt, bevor sich der Zervikalanschlag in Anlage an der Zervikalöffnung befindet. Sobald diese Anlagestellung erreicht ist, wird das Rohr auf dem Kolben nach außen gezogen, so daß das Gerät im Inneren der Uterushöhlung verbleibt, ohne dort Schädigungen hervorrufen zu können. Die im Rohr entgegen ihrer elastischen Vorspannung zusammengepreßten Zweige des Geräts spreizen sich beim Auswerfen elastisch auf und kommen zu einer das Gerät halternden Anlage an der Innenwand des Uterus.

Auch aus US-A-4 117 838 und US-A-3 937 217 sind bereits Geräte mit elastisch verformbaren Haltern in Form von offenen Bögen bzw. einer tropfenförmigen Schleife bekannt. Diesen bekannten Formen ist die erfindungsgemäß vorgesehene Form einer geschlossenen Raute mit ausbiegbaren Armen im Hinblick auf die angestrebte Wirkung überlegen.

Die elastisch ausbiegbaren Arme des Halters können zweckmäßigerweise aus geradlinigen oder gewellten Abschnitten bestehen, die zusammen eine Raute bilden. In vorteilhafter Weiterbildung können auch vier Arme vorgesehen sein, die zusammen zwei um 90° um die gemeinsame Mittelachse verdrehte Rauten bilden.

Verschiedene Kunststoffe eignen sich für die Herstellung des erfindungsgemäßen Gerätes. Bevorzugt wird jedoch die Verwendung von Polyhexamethylenadipinamid (Nylon).

Um beim Einführen oder Benutzen des Geräts störende Querschnittsänderungen der Zweige oder das Abstehen von zu Verletzungen Anlaß gebenden Drahtenden sicher zu vermeiden, kann bei einer weiteren vorteilhaften Ausgestaltung der Erfindung jeweils wenigstens ein Ende der Kupferdrahtwicklungen in einer Vertiefung des zugehörigen Zweiges aufgenommen sein. Zusätzlich oder statt dessen können die Zweige in Abstufungen zu einem kleineren Querschnitt übergehen und die Kupferdrahtwicklungen in dem Bereich mit kleinerem Querschnitt bündig mit den Bereichen mit größerem Querschnitt aufgebracht werden.

Um die beim Einführen des Geräts vorangehenden verdickten Köpfe möglichst störungsfrei einführen und in ihre Benutzungslage bringen zu können, werden sie nach einem weiteren Vorschlag der Erfindung zweckmäßigerweise an ihrer von den formschlüssig ineinanderfügbaren Teilen wegweisenden Außenseite löffelförmig ausgebildet. Statt dessen können sie auch mit einem weich abgerundeten, zum Halter des Geräts hinweisenden Haken versehen sein, der einerseits die weiche Kontur beim Einführen und Anliegen an der Uteruswand gewährleistet, andererseits zur Verhinderung eines unbeabsichtigten Herausgleitens des Geräts aus dem Uterus beiträgt.

Damit beim Einführen des Geräts nicht die vordere Kante des Auswerferrohrs vorangeht, sondern die abgerundeten Köpfe, werden diese vorzugsweise so ausgebildet, daß sie in formschlüssig ineinandergefügtem Zustand einen Außendurchmesser aufweisen, der größer ist als der Innendurchmesser des Rohrs. Gleichzeitig kann die Außenfläche des Rohrs an dem dem Gerät zugewandten Ende nach innen abgeschrägt sein, so daß einerseits die Köpfe gut auf dieser Vorderkante des Rohrs aufliegen und andererseits keine scharfe Außenkante an der Wand des Zervikalkanals entlanggleiten kann.

Die Schwergängigkeit zwischen Rohr und Kolben wird bei einer sehr einfachen Ausführungsform dadurch verwirklicht, daß das Rohr wenigstens eine Stelle aufweist, an der sein Innendurchmesser in unbelastetem Zustand kleiner ist als der Außendurchmesser des Kolbens. Infolge der Elastizität des Rohrs ist ein schwergängiges Durchschieben des Kolbens durch diese verengte Stelle ohne weiteres möglich.

Anhand der Figuren werden Ausführungsbeispiele der Erfindung näher erläutert. Es zeigt

Fig. 1 eine teilweise geschnittene Seitenansicht einer Ausführungsform der Vorrichtung während des Auswerfens des Geräts aus dem Auswerferrohr,

Fig. 2 eine der Fig. 1 entsprechende Seitenansicht bei mit Ausnahme der Köpfe vollständig in das Rohr eingeführtem Gerät,

Fig. 3 eine schematische Darstellung eines Uterus in einem längs einer Frontalebene geführten Schnitt mit der in den Uterus eingeführten Vorrichtung gemäß Fig. 2,

Fig. 4 eine der Fig. 3 entsprechende Darstellung eines Uterus mit einem darin eingesetzten erfindungsgemäßen Gerät,

Fig. 5 eine schematische Teilansicht einer Ausführungsform des erfindungsgemäßen Geräts, das längs seiner Mittelachse X-X abgeschnitten ist,

Fig. 6 eine schematische Teilansicht einer anderen Ausführungsform des freien Endes eines Zweiges,

Fig. 7 einen Schnitt längs der Linie 7-7 in den Fig. 5 und 6,

Fig. 8 einen Schnitt längs der Linie 8-8 in Fig. 6,

Fig. 9 einen Schnitt längs der Linie 9-9 in Fig. 5,

Fig. 10 einen Teilschnitt durch das vordere Ende des Auswerferrohres,

Fig. 11 einen der Fig. 10 entsprechenden Teilschnitt mit in das Auswerferrohr eingeführtem Gerät,

Fig. 12 eine Stirnansicht der in Fig. 11 gezeigten Teile,

Fig. 13 eine schematische Seitenansicht einer Ausführungsform des Halters des Gerätes und

Fig. 14 eine schematische Seitenansicht des Halters einer anderen Ausführungsform des Gerätes.

Für gleiche oder entsprechende Teile sind in allen Figuren die gleichen Bezugszahlen verwendet.

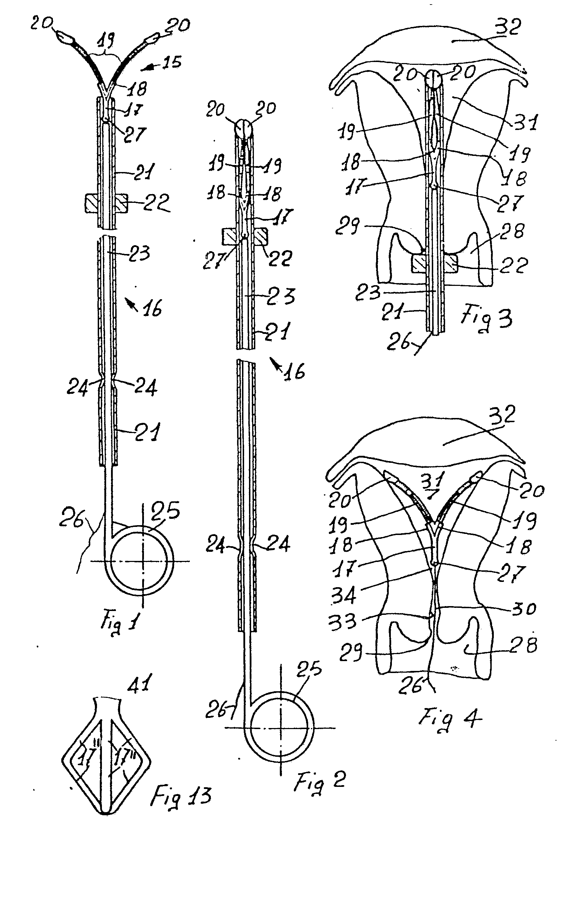

Die erfindungsgemäße Vorrichtung besteht, wie aus Fig. 1 ersichtlich ist, aus einem in die Uterushöhlung einzubringenden Gerät 15 und einem Auswerfer 16. In der Stellung der Fig. 1 ist der den unteren Teil des Geräts bildende Halter 17 in das Auswerferrohr 21 eingefahren. Vom Halter 17 gehen zwei bogenförmige Zweige 18 aus, die teilweise mit Kupferdrahtwicklungen 19 bis zu den an ihren Enden sitzenden verdickten Köpfen 20 umwickelt sind. Die Köpfe sind an ihrer Außenseite löffelförmig abgerundet.

Der Auswerfer 16 weist ein zylindrisches und elastisch nachgiebiges Rohr 21, einen Anschlagflansch 22, der längs des Rohres 21 einstellbar ist, einen Kolben 23, dessen Außendurchmesser geringer ist als der Innendurchmesser des Rohres 21 und der durch zwei einander gegenüberliegende, auf dem letzteren vorgesehene Einziehungen 24 schwergängig im Rohr 21 verschiebbar ist, und einen Handgriff 25 am Ende des Kolbens 23 auf. Das Rohr 21 kann auf seiner Außenseite zur genauen Positionierung des Anschlagflansches 22 eine nicht dargestellte Skaleneinteilung tragen, die derjenigen eines Hysterometers entspricht. Der Anschlagflansch 22 kann auch als Zervikalanschlag bezeichnet werden.

Die Einführung des Geräts 15 in den Auswerfer 16 erfolgt mittels eines Nylonfadens 26, dessen eines Ende an einer Öffnung 27 des Halters 17 angeknüpft und durch das Rohr 21 aus dessen nahe dem Handgriff 25 gelegenem Ende nach außen geführt ist. Durch Zugausübung auf diesen Nylonfaden 26 wird das Gerät 15 in die in Fig. 2 gezeigte Stellung gebracht. Dabei wird gleichzeitig der Kolben 23 im Rohr 21 nach unten verschoben. Der Anschlagflansch 22 wird vor Benutzung der Vorrichtung auf eine Entfernung vom oberen Ende der Köpfe 20 eingestellt, die etwa 1 cm geringer ist als die Tiefe des Uterus. Dadurch ist eine sichere Einführung des Geräts 15 in den Uterus mittels des Auswerfers 16 gewährleistet.

Zur Einführung des Geräts 15 in den Uterus wird das Rohr 21 in seinem unteren Endbereich mit zwei Fingern ergriffen und gegen den Kolben 23 gedrückt. Sodann wird der obere Teil des Auswerfers 16 in die Scheide 28 eingeführt, wobei die Köpfe 20 zum Eindringen in die äußere Zervikalöffnung 29 gebracht werden. Sodann wird der Auswerfer 16 mittels einer hin- und hergehenden spiraligen Bewegung in den Zervikalkanal 30 und anschließend in die Uterushöhlung 31 so weit eingeführt, bis der Zervikalanschlag 22 mit der Öffnung 29 in Berührung kommt (siehe Fig. 3).

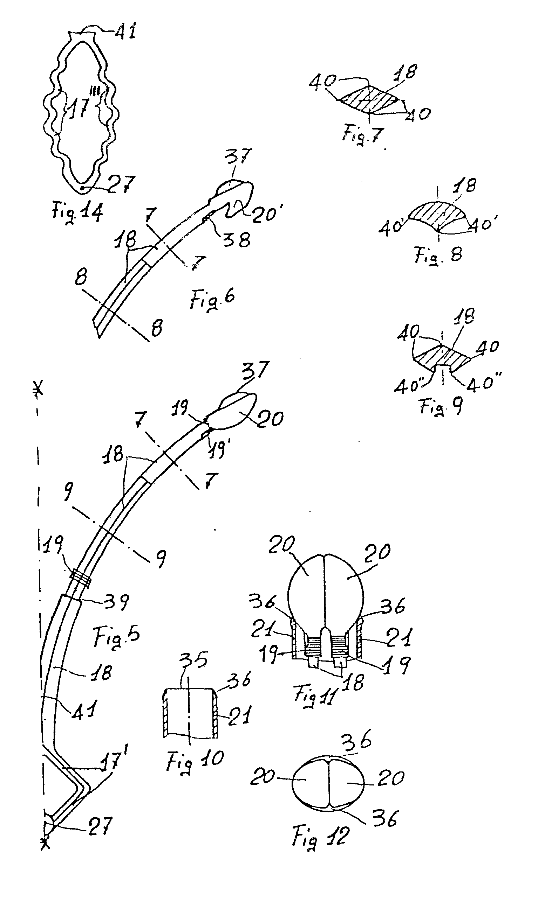

Während dieses Einführens besteht weder die Gefahr einer Perforation des Uterusbodens 32 noch die Gefahr eines Abriebs oder von Abschürfungen des Endometriums 33 längs des Kanals 30 und der inneren Zervikalöffnung 34, weil einerseits die Eindringtiefe des Auswerfers 16 vorher mit einem hinreichenden Sicherheitsgrad durch die Einstellung des Zervikalanschlags 22 festgelegt wurde und andererseits das Rohr 21 in seinem oberen Teil gemäß Fig. 10 dank der schrägen Wand 36 eine zur Öffnung 35 hin sich verringernde Dicke aufweist. Daher ist die Dicke des Rohrs 21 in der Nähe der Öffnung 35 zu vernachlässigen, und das Rohrende wird überdies von den Erweiterungen der löffelförmigen Köpfe 20 verdeckt, die der Öffnung 35 eine ovale Form geben, indem sie bewirken, daß sich diese Öffnung an ihre äußere Oberfläche anlegt (siehe Fig. 11 und 12). Wesentlich ist auch, daß die dem Auswerfer 16 erteilte hin- und hergehende spiralige Bewegung keine Verschiebung des einen Kopfes 20 gegenüber dem anderen zur Folge haben kann, da gemäß Fig. 5 der Kopf 20 mit einer Erhebung 37 versehen ist, die sich in eine geeignete Vertiefung in dem anderen nicht dargestellten Kopf einpaßt. Diese Ausbildung der Köpfe verhindert jedes Einzwicken und sich daraus ergebende Schädigungen des Endometriums 33 während des Einführens des Auswerfers 16 in den Uterus.

Gemäß Fig. 5 und 6 weisen die Zweige 18 in der Nähe des an der Außenseite löffelförmig ausgebildeten Kopfes 20 bzw. des hakenförmig ausgebildeten Kopfes 20' eine Einziehung 38 (Fig.6) für die Aufnahme der Endteile 19' (Fig. 5) der Kupferdrahtwicklung 19 auf. Dadurch wird vermieden, daß die Endteile 19' eine Verdickung der Kupferdrahtwicklung 19 verursachen.

Die Kupferdrahtwicklung 19 endet unten gegen eine Abstufung 39 des Zweiges 18 (Fig. 5), so daß sich an dieser Stelle ein bündiger Übergang zwischen dem nicht bedeckten bogenförmigen Zweig 18 und der Kupferdrahtwicklung 19 ergibt.

Sobald der Auswerfer 16 gemäß Fig. 3 in den Uterushohlraum 31 eingeführt ist, erfolgt die Positionierung des Geräts 15 dadurch, daß der Kolben 23 festgehalten und das Rohr 21 gleitend nach unten bewegt wird, so daß die Köpfe 20 infolge der Vorspannung der Zweige 18 sich so weit voneinander entfernen, bis sie die in Fig. 4 dargestellte Stellung annehmen. Dies ist der Fall, sobald das obere Ende des Rohres 21 das Ende des Kolbens 23 erreicht hat, so daß das Gerät 15 vollständig vom Auswerfer 16 freigegeben wird.

Sodann werden gleichzeitig das Rohr 21 und der Kolben 23 aus dem Zervikalkanal 30 und der Scheide 28 herausgezogen, wobei der Nylonfaden 26 im Zervikalkanal 30 und der Scheide 28 verbleibt und aus dieser nach außen steht. Durch Zugausübung auf den Nylonfaden 26 kann das Gerät 15 jederzeit aus dem Uterus und der Scheide nach außen gezogen werden.

Sobald das Gerät 15 in seine endgültige Lage gemäß Fig. 4 gebracht ist, bleiben die Krümmungsradien der Zweige 18 infolge der an denselben angebrachten Verstärkungsrippen 40, 40', 40'', (Fig. 7, 8, 9) weitgehend stabil. Diese Rippen stellen keine Gefahr für die Unversehrtheit des Endometriums 33 dar, da sie mit der Kupferdrahtwicklung 19 umwickelt sind.

Bei der Ausführungsform gemäß Fig. 5 weist der Halter 17' zwei elastisch ausbiegbare Arme in Form einer Raute auf. Die beiden Arme des Halters 17' einerseits und die beiden Zweige 18 andererseits vereinigen sich in einem gemeinsamen Halterabschnitt 41. Eine Diagonale des rautenförmigen Halters 17' fällt mit der Mittelachse X-X des Geräts 15 zusammen.

Durch die elastische Ausbildung des Halters 17' wird die durch Uteruskontraktionen hervorgerufene Ausstoßwirkung aufgefangen. Der Halter 17' kann sich unter der Einwirkung dieser Kontraktionen verlängern und wieder in seine Normallage zurückkehren, sobald eine Kontraktion beendet ist. Ein spontanes und ungewolltes Ausstoßen des Geräts 15 aus der Uterushöhlung wird damit im Zusammenspiel mit der stabilen Spreizwirkung der Zweige 18 sicher verhindert.

Eine andere Ausführungsform des Halters 17'' ist in Fig. 13 dargestellt. Hier besteht der Halter aus vier Armen, die in Form von zwei gegenseitig um 90° um die gemeinsame Mittelachse X-X verdrehten Rauten angeordnet sind. Dadurch wird die Haltewirkung im Zervikalkanal noch verstärkt.

Eine besonders gute Elastizität besitzt die in Fig. 14 gezeigte Ausführungsform des Halters 17'''. Bei dieser Ausführungsform verlaufen die ebenfalls etwa rautenförmigen Arme des Halters nicht geradlinig, sondern gewellt.

**Patentansprüche**

1. Vorrichtung zur intrauterinen Empfängnisverhütung, mit einem aus einem elastischen Kunststoff bestehenden, Y-förmigen Gerät (15), das einen eine Mittelachse besitzenden Halter (17) und zwei von diesem ausgehende, bogenförmig aufgespreizte Zweige (18) aufweist, die je eine Kupferdrahtwicklung (19) tragen und in einem verdickten Kopf (20, 20') enden, und mit einem Auswerfer, der ein Rohr (21) und einen im Rohr teleskopartig verschiebbaren, an einem Ende einen Handgriff (25) tragenden Kolben (23) aufweist, wobei das Gerät (15) wenigstens teilweise in das Rohr (21) einführbar ist, das Rohr (21) schwergängig auf dem Kolben (23) sitzt und auf seiner Außenseite einen Anschlagflansch (22) trägt und das Rohr (21) elastisch verformbar ist, dadurch gekennzeichnet, daß der Halter (17) in Richtung seiner Mittelachse elastisch verformbar ist, indem er in sich mindestens zwei bezüglich der Mittelachse (X-X) elastisch ausbiegbare Arme (17') aufweist, die zusammen eine Raute bilden, deren eine Diagonale mit der Mittelachse (X-X) zusammenfällt, daß jeder Zweig (18) mindestens eine die Aufspreizstellung stabilisierende Verstärkungsrippe (40, 40', 40") trägt und daß die beiden Köpfe (20) formschlüssig ineinanderfügbare Teile (z. B. 37) aufweisen.

2. Vorrichtung nach Anspruch 1, dadurch gekennzeichnet, daß die Arme (17''') gewellt verlaufen.

3. Vorrichtung nach Anspruch 1 oder 2, dadurch gekennzeichnet, daß vier Arme (17") vorgesehen sind, die zusammen zwei um 90° um die gemeinsame Mittelachse (X-X) verdrehte Rauten bilden.

4. Vorrichtung nach einem der vorangehenden Ansprüche, dadurch gekennzeichnet, daß das Gerät (15) aus Polyhexamethylenadipinamid (Nylon) hergestellt ist.

5. Vorrichtung nach einem der vorangehenden Ansprüche, dadurch gekennzeichnet, daß jeweils wenigstens ein Ende (19') der Kupferdrahtwicklungen (19) in einer Vertiefung (38) des zugehörigen Zweiges (18) aufgenommen ist.

6. Vorrichtung nach einem der vorangehenden Ansprüche, dadurch gekennzeichnet, daß die Zweige (18) in Abstufungen (39) zu einem kleineren Querschnitt übergehen und daß die Kupferdrahtwicklungen (19) in dem Bereich mit kleinerem Querschnitt bündig mit den Bereichen mit größerem Querschnitt aufgebracht sind.

7. Vorrichtung nach einem der vorangehenden Ansprüche, dadurch gekennzeichnet, daß die verdickten Köpfe (20, 20') an ihrer von den formschlüssig ineinandergefügten Teilen (37) wegweisenden Außenseite löffelförmig oder hakenförmig ausgebildet sind.

8. Vorrichtung nach einem der vorangehenden Ansprüche, dadurch gekennzeichnet, daß die beiden Köpfe (20) in formschlüssig ineinandergefügtem Zustand einen Außendurchmesser aufweisen, der größer ist als der Innendurchmesser des Rohrs (21).

9. Vorrichtung nach Anspruch 8, dadurch gekennzeichnet, daß die Außenfläche des Rohrs (21) an dem dem Gerät (15) zugewandten Ende nach innen abgeschrägt ist.

10. Vorrichtung nach einem der vorangehenden Ansprüche, dadurch gekennzeichnet, daß das Rohr (21) wenigstens eine Stelle (24) aufweist, an der sein Innendurchmesser in unbelastetem Zustand kleiner ist als der Außendurchmesser des Kolbens (23).

**Claims**

1. Apparatus for intrauterine contraception with an Y-shaped device (15) made of resilient plastic comprising a holding means (17) having a central axis, and two branches (18) spreading apart from said holding means in an arcuate way, each carrying a copper-wire winding (19) and terminating in a thickened head (20, 20'), and with an ejection means having a tube (21) and a piston (23) slidably telescoping in the tube and carrying a handle (25) on one end, the device (15) being adapted for being at least partially inserted into the tube (21), said tube (21) sitting on said piston (23) with dragging fit and having on its outer face a stop flange (22), and said tube (21) being resilient, characterized in that the holding means (17) is resilient along its central axis, having at least two arms (17') adapted for being bent out resiliently with respect to the central axis (X-X) and forming together a lozenge having one of its diagonals coinciding with the central axis (X-X), that each branch (18) carries at least one reinforcing rib (40, 40', 40") stabilizing the spreading position, and that the two heads (20) comprise sections (e. g. 37) form-fitting into each other.

2. Apparatus in accordance with claim 1, characterized in that arms (17''') have an undulated shape.

3. Apparatus in accordance with claim 1 or 2, characterized in that four arms (17") are provided, forming together two lozenges mutually offset by 90° around the common central axis (X-X).

4. Apparatus in accordance with one of the preceding claims, characterized in that the device (15) is made of polyhexamethylene adipinamide (Nylon).

5. Apparatus in accordance with one of the preceding claims, characterized in that at least one end (19') of each of the copper-wire windings (19) is lodged in a recess (38) of the associated branch (18).

6. Apparatus in accordance with one of the preceding claims, characterized in that the branches (18) are graduating by steps (39) into portions with reduced cross-section and that the copper-wire windings (19) are applied on the portion with reduced cross-section in such a way as to be flush with the portions having the larger cross-section.

7. Apparatus in accordance with one of the preceding claims, characterized in that thickened heads (20, 20') are spoon-shaped or hook-shaped in their outer faces pointing away from the sections (37) formfitting into each other.

8. Apparatus in accordance with one of the preceding claims, characterized in that the two heads (20), when form-fitted into each other, have an outside diameter larger than the inside diameter of the tube (21).

9. Apparatus in accordance with claim 8, characterized in that the outer face of said tube (21) is inwardly tapered on its end facing the device (15).

10. Apparatus in accordance with one of the preceding claims, characterized in that said tube (21) has at least one area (24) where its inside diameter in unloaded condition is smaller than the outside diameter of said piston (23).

## Revendications

1. Dispositif anticonceptionnel intime, avec un appareil en forme de Y (15) constitué d'une matière plastique élastique, qui comporte un support (17) avec un axe médian et deux branches (18) partant de ce support et s'écartant en forme d'arcs, ces branches portant chacune un enroulement de fil de cuivre (19) et se terminant par une ête épaisse (20, 20'), et avec un éjecteur comportant un tube (21) et un piston (23) susceptible de se déplacer télescopiquement dans ce tube et portant à son extrémité une poignée (25), l'appareil (15) étant susceptible d'être introduit au moins partiellement dans le tube (21), ce tube (21) étant placé à frottement dur sur le piston (23) et portant sur sa face externe une collerette de butée (22) tandis que le tube (21) est susceptible de se déformer élastiquement, dispositif caractérisé en ce que le support (17) est susceptible de se déformer élastiquement en direction de son axe médian, du fait qu'il comporte au moins deux bras (17') susceptibles de fléchir élastiquement vers l'extérieur par rapport à l'axe médian (X-X), ces bras formant, ensemble, un losange dont une diagonale coïncide avec l'axe médian (X-X), chaque branche (18) portant au moins une nervure de renforcement (40, 40', 40") stabilisant la position d'écartement, tandis que les deux têtes (20) de ces branches comportent des parties (par exemple 37) se joignant par interpénétration de forme.

2. Dispositif selon la revendication 1, caractérisé en ce que les bras (17''') sont ondulés.

3. Dispositif selon la revendication 1 ou 2, caractérisé en ce qu'il est prévu quatre bras (17") formant ensemble deux losanges décalés l'un de l'autre de 90° autour de l'axe médian commun (X-X).

4. Dispositif selon l'une des revendications précédentes, caractérisé en ce que l'appareil (15) est réalisé en polyhexaméthylène adipinamide (nylon).

5. Dispositif selon l'une des revendications précédentes, caractérisé en ce que, au moins une extrémité (19') de chacun des enroulements de fil de cuivre (19) est logée dans une cavité (38) de la branche (18) associée.

6. Dispositif selon l'une des revendications précédentes, caractérisé en ce que la section transversale des branches (18) est réduite par des épaulements (39) et que les enroulements de fil de cuivre (19) sont rapportés sur la partie de section transversale plus réduite sans solution de continuité avec les parties de section transversale plus importante.

7. Dispositif selon l'une des revendications précédentes, caractérisé en ce que les têtes plus épaisses (20, 20') présentent sur leurs faces externes, opposées à leurs parties (37) jointes l'une à l'autre par interpénétration de forme, une forme de cuillère ou de crochet.

8. Dispositif selon l'une des revendications précédentes, caractérisé en ce que les deux têtes (20) lorsqu'elles sont jointes l'une à l'autre par interpénétration de forme, présentent un diamètre externe supérieur au diamètre interne du tube (21).

9. Dispositif selon la revendication 8, caractérisé en ce que la surface externe du tube (21) est chanfreinée vers l'intérieur à son extrémité tournée vers l'appareil (15).

10. Dispositif selon l'une des revendications précédentes, caractérisé en ce que le tube (21) comporte au moins un emplacement (24) où son diamètre interne, à l'état libre, est inférieur au diamètre externe du piston (23).

Fig 1

Fig 13

Fig 2

Fig 3

Fig 4

Fig 14

Fig.6

Fig.7

Fig. 8

Fig. 9

Fig.5

Fig 10

Fig 11

Fig 12